# EUROPÄISCHE PATENTSCHRIFT

(11) **EP 1 466 880 B1**
(45) Veröffentlichungstag und Bekanntmachung des Hinweises auf die Patenterteilung: **04.06.2014**
(21) Anmeldenummer: 04008512.8
(22) Anmeldetag: 08.04.2004
(51) Int. Cl.: A61L 9/16, B01D 53/75, B09B 3/00, C05F 17/02

(54) **Verfahren und Vorrichtung zur biologisch mechanischen Behandlung von organischem Material**
Process and apparatus for the biomechanical processing of organic material
Procédé et appareil pour le traitement biomechanique de matière organique

(30) Priorität: 09.04.2003 AT 25103 U
(43) Veröffentlichungstag der Anmeldung: 13.10.2004
(73) Patentinhaber: Lübke, Aurel, 4084 St. Agatha (AT); New Earth Solutions Group Limited, Ebblake Industrial Estate Verwood Dorset BH31 6AT (GB)
(72) Erfinder: Lübke, Aurel, 4084 St. Agatha (AT)
(74) Vertreter: Secklehner, Günter

(56) Entgegenhaltungen:
- EP-A- 1 386 675
- WO-A-00/26337
- US-A- 3 895 916

## Beschreibung

Die Erfindung betrifft ein Verfahren zur biologisch mechanischen Behandlung von organischem Material in einem Raum mit den Merkmalen des Oberbegriffes des Anspruches 1 und eine Vorrichtung zur Durchführung des Verfahrens mit den Merkmalen des Oberbegriffes des ersten Vorrichtungsanspruches 14.

Kompostmieten werden auf befestigtem Boden in Längsrichtung parallel zueinander über Belüftungskanäle aufgesetzt und werden bei Abtrocknung oder beim Umsetzen gegen die Staubentwicklung, mit Beregnungseinrichtungen feucht gehalten.

Bei diesem Verfahren tritt eine hohe Geruchs- und Schadstoffbelastung der Umgebung auf; besonders beim Bearbeiten mit Umsetzmaschinen ist der Emissionsanstieg sehr nachteilig für dichter besiedeltes Gebiet.

Weiters tritt bei den im Stand der Technik bekannten Kompostiersystemen wie aus den Dokumenten EP 1386675 A2 oder US 3895916 bekannt, der Nachteil auf, dass beim Umsetzen des Materials der Kompostmieten mit einem üblicherweise durch eine Person bedienten Umwälzgerät durch die aufgewirbelten Staub- und Schmutzpartikel und die mit Wasserdampf gesättigte Luft zumindest im Nahbereich der Behandlungsstelle schlechte Sichtbedingungen und gegebenenfalls eine erhöhte Gesundheitsbelastung für die Person bestehen. Insbesondere bei Nebelaufkommen in dem im Wesentlichen abgeschlossenen Raum hat dies negative Auswirkungen auf die Arbeitseffizienz.

Aufgabe der Erfindung ist es, ein Verfahren zur Kompostierung und zur Umsetzung der Kompostmieten anzugeben mit dem die angegebenen Nachteile, vor Allem die unzulässig hohe Geruchs- und Staubbelastung weitgehend vermieden werden und die Mittel für die Durchführung des Verfahrens zu schaffen, mit denen dieses Ziel erreicht wird.

Diese Aufgabe wird bei einem Verfahren der eingangs genannten Gattung mit den Merkmalen des kennzeichnenden Teiles des Anspruches 1 gelöst und für die Vorrichtungsmittel der eingangs genannten Gattung mit den Merkmalen des kennzeichnenden Teiles des ersten Vorrichtungsanspruches gelöst.

Die Unteransprüche betreffen vorteilhafte und wichtige Fortbildungen der Erfindung und sind ebenso wie Patentanspruch 1 und der erste Vorrichtungsanspruch gleichzeitig ein Teil der Beschreibung der Erfindung.

Die sich aus der erfindungsgemäßen Lösung gemäß den Ansprüchen 1 bzw. 4 ergebenden Vorteile liegen vor allem darin, dass mit einem derartigen Verfahren das Raumklima im Bereich der Kompostmieten dahingehend verbessert werden kann, dass aufgrund der kontinuierlichen Durchlüftung des Raumes durch Zufuhr von schmutzfreier Zuluft und der Abfuhr von mit Partikeln und/oder Dampf kontaminierter Raumluft neben der Verhinderung von Nebelbildung auch der Anteil von Schmutzpartikeln in der Luft gering gehalten werden kann.

Im Anspruch 2 ist eine zweckmäßige Maßnahme zur Schaffung des notwendigen Raumklimas dargelegt.

Die Merkmale des Anspruches 3 sind aufgrund der somit geringen Belastung für die Außenumgebung durch Filterung der Emissionen von Vorteil.

Die im Anspruch 5 beschriebenen Maßnahmen sind vorteilhaft, da die Raumluft durch die eingebrachte Zuluft zumindest im vor dem Umwälzgerät liegenden Bereich unterhalb der erforderlichen Grenzwerte gehalten werden kann. Für eine das Umwälzgerät bedienende Person herrscht in Umwälzrichtung somit eine freie Sicht und es besteht keine erhöhte, gesundheitliche Belastung. Durch Abfuhr bzw. Absaugung der Luft des hinter dem Umwälzgerät liegenden Raumes wird diese gezielt und in möglichst kleinem Volumen abtransportiert, sodass eine nachgeschaltete Abluftbehandlungsanlage ein geringeres Abluftvolumen verarbeiten muss und diese kompakt und kostengünstig aufgebaut sein kann.

Durch den Anspruch 6 wiedergegebene Maßnahme der Erzeugung eines Luftstroms kann in einfacher Weise eine kontinuierliche Verfrachtung von Nebel bzw. Schmutzpartikeln in den hinter dem Umwälzgerät liegenden Bereich und in weiterer Folge in die Außenumgebung erreicht werden.

Durch einen Verfahrensschritt gemäß Anspruch 7 kann in einfacher Weise eine die gesamte Hallenlänge bzw. Raumlänge durchlaufende Luftströmung erzeugt werden.

Durch die in Anspruch 8 beschriebene Maßnahme ist eine rasche und auf die kontaminierten Bereiche der Luft des Raumes beschränkte Abfuhr der Abluft möglich, indem die entsprechend in diesen Bereichen angesiedelten Luftdurchlässe aktiviert werden. Somit ist ein effektiver Austausch von geringen Luftmengen möglich.

Durch die in Anspruch 9 beschriebene Maßnahme kann in vorteilhafter Weise die in der Abluft vorhandene Wärmeenergie genutzt werden, wobei z.B. durch Vorwärmung der in den Raum einzubringenden Zuluft mit dieser Wärmeenergie eine Nebelbildung im Raum verhindert werden kann.

Die Maßnahme nach Anspruch 10 ist von Vorteil, da nach Festlegung des Parameters der Umwälzrichtung durch die Regelungseinrichtung die optimale Belüftung des Raumes automatisiert erfolgen kann.

Die in Anspruch 11 beschriebene Maßnahme ist von Vorteil, da durch die gereinigte Abluft die Schadstoffemissionen an die äußere Umwelt gering gehalten bzw. verhindert werden können.

Ebenso ist die Maßnahme nach Anspruch 12 vorteilhaft, da in Abhängigkeit der Kapazität der Abluftbehandlungsanlage bzw. des Biofilters bei zu hohem Abluftvolumen die überschüssige Abluft unbehandelt zurückgespeist werden kann. Beispielsweise ist es möglich, dass die zurückgespeiste Abluft in ein oder mehrere diskrete Räume unterschiedlicher Hallen eingebracht wird, in denen zu diesem Zeitpunkt keine Behandlung von Material stattfindet, sodass sich Verunreinigungen in der Luft am Boden absetzten können. Die Abluftbehandlungsanlage kann somit mit einer geringen bzw. der geringst möglichen Abluftmenge beaufschlagt werden, was einen kompakten und kostengünstigen Aufbau ermöglicht.

Im Anspruch 13 ist eine Maßnahme zur Steigerung der Effizienz des Verfahrens beschrieben.

Die Vorteile der Vorrichtungsansprüche 14 bis 25 ergeben sich aus der nachstehenden Beschreibung bzw. korrespondieren mit den vorstehend beschriebenen Vorteilen.

Die Erfindung wird anhand von schematischen Zeichnungen zu drei Ausführungsbeispielen beschrieben.

Es zeigen:
- Fig. 1: die Vorrichtungen zur Durchführung des Verfahrens in einem Raum, bei der die Raumwände und das Dach in Sichtrichtung weggelassen sind, in Schrägansicht, schematisch;
- Fig. 2: ein zweites Ausführungsbeispiel der Vorrichtung zur Durchführung des Verfahrens in einem Raum, dargestellt in Schrägansicht und teilweise in den Innenraum der Halle freilegender Bruchdarstellung, schematisch.

In einer dargestellten Halle 1, die zwei Raumeinheiten bildet, mit an einer der Stirnseiten 2 angebrachten Rolltoren 3, werden auf dem befestigten Hallenboden 4 mehrere Kompostmieten 5 parallel zueinander dicht an dicht nebeneinander aufgesetzt.

Unter den Kompostmieten 5 verlaufen mittig in Längsrichtung 6 der Halle 1 Abluftkanäle 7, durch die Abluft abgesaugt wird.

Es ist für jede Kompostmiete 5 an einem Ende der Halle 1 ein Abluftgebläse 8 angeordnet um jeweils auch nur eine bestimmte Kompostmiete 5 abzusaugen und alle Abluftgebläse 8 sind mit einem seitlich neben der Halle 1 vorgesehenen Biofilter 9 verbunden, in das die Abluft geleitet wird.

Oberhalb der Kompostmieten 5 sind in Abständen verteilt Beregnungsvonichtungen 10 angeordnet, mit denen die Kompostmieten 5 beregnet werden können.

In den Kompostmieten 5 sind Temperaturfühler 11 eingesetzt, von denen Daten per Funk an die Empfangs-Sendeeinrichtung 23 einer Regelungseinrichtung 12 übertragen werden, die in einer Steuerzentrale 13, die vor einer der Stirnseiten 2 der Halle 1 aufgebaut ist und von der aus Regelsignale per Funk von der Empfangs-Sendeeinrichtung 23 an das entsprechende Abluftgebläse 8 gesendet werden, wenn die zulässige Mietentemperatur überschritten wird.

Um das gefahrlose Betreten der Halle 1 zu gestatten oder um übermäßige Schadstoffanreicherung in der Hallenluft herabzusetzen, ist an einer Stirnseite 2 eine Absaugung 15 vorgesehen, für die von einer Zuluftöffnung 14 im Hallendach 16 der Halle 1 her in den Hallenraum Zuluft eingespeist wird.

Im Austritt der Absaugung 15 ist eine chemische Behandlungsstufe mit einem Zerstäuber angeordnet, mit dem die Abluft behandelt wird und Geruchs- und Staubbelastung in der ins Freie gelangenden Abluft beseitigt wird

Unter dem Hallendach 16 ist zwischen den Kompostmieten 5 eine Umlufteinrichtung 17 aufgehängt, bei der ein Umluftrohr 18 mit weitem Lumen, zur Aufnahme der beim Umsetzen einer Kompostmiete 5 entwickelten staub- und schadstoffbelasteten Luft vorgesehen ist, wobei je nach Umluftrichtung 19 einer der beiden Förderlüftersätze 20 die Luft einstellbar axial und nach unten gerichtet hindurchsaugt.

Die Luft wird mit einem, beiden oder keinem der vorgesehenen Beregnern 21 nach Bedarf befeuchtet und gekühlt und dann von einem mittig im Umluftrohr 18 angebrachten Zerstäuber 22 mit Zusatzstoffen versetzt.

Der auftretende Unterdruck im Umluftrohr 18 unterdrückt eine verstärkte Nebelbildung in der austretenden Umluft.

Die Umluftrichtung 19 ist dabei der Fahrtrichtung der Umsetzmaschine entgegengerichtet.

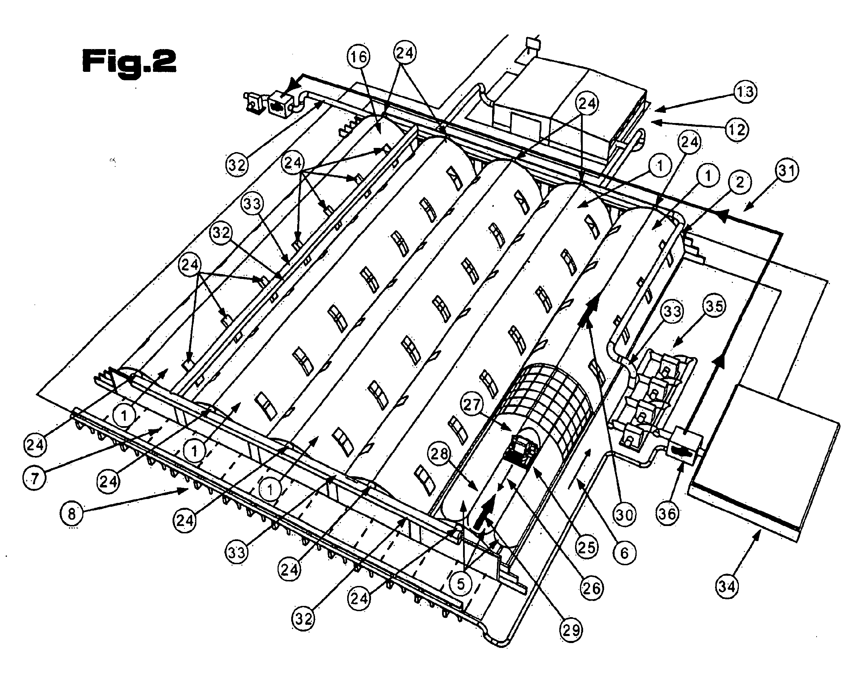

In der Fig. 2 ist eine weitere mögliche Ausführungsvariante der Erfindung dargestellt, wobei fünf nebeneinander stehende Hallen 1 gezeigt sind. Selbstverständlich ist eine beliebige Anzahl von Hallen 1 bzw. eine abweichende Aufstellung dieser Hallen 1 zueinander möglich. In jeder der Hallen 1 sind mehrere Kompostmieten 5 im Wesentlichen parallel in Längsrichtung der Halle 1 angeordnet. Auch die Anzahl der in einer Halle 1 befindlichen Kompostmieten 5 ist nicht auf die dargestellte Ausführungsvariante beschränkt, sondern es können in Abhängigkeit der Abmessungen der Halle 1, insbesondere einer Hallenbreite, die sich daraus ergebende Anzahl von Mieten angeordnet sein. Beispielsweise hat sich eine Breite der Halle 1 von in etwa 10 bis 15 m und eine Anzahl von etwa 2 bis 4 nebeneinander verlaufender Mieten 5 als zweckmäßig erwiesen.

In der stark vereinfachten und schematisch dargestellten Ausführungsvariante gemäß Fig. 2 ist die gegebenenfalls vorhandene Absaugung 15, die Umlufteinrichtung 17 und der Beregner 21 der Übersichtlichkeit halber nicht dargestellt. Diese Komponenten können bei den zur Fig. 2 und 3 beschriebenen Ausführungsbeispielen selbstverständlich vorhanden sein bzw. zur Durchführung des erfindungsgemäßen Verfahrens ausbildet sein.

Im Ausführungsbeispiel gemäß Fig. 2 soll das Prinzip der Luftumwälzung beim erfindungsgemäßen Verfahren sowie die hierzu erforderliche bauliche Ausgestaltung beschrieben werden. Beim Verfahren zur biologisch-mechanischen Behandlung des organischen Materials, vorzugsweise mit Absaugen von Abluft unterhalb des Materials, erfolgt die Behandlung des Materials durch Umsetzen oder dgl. in einem im Wesentlichen geschlossenen Raum. Dieser Raum befindet sich beispielsweise innerhalb der Halle 1, wobei unter dem Begriff "Halle" sämtliche, diskrete und im Wesentlichen in sich geschlossene Raumeinheiten, unabhängig von deren Bausubstanz oder dgl., fallen. Einzelne Hallen 1 mit entsprechenden Räumen können beispielsweise Einzelsektionen einer größeren Halle bilden, die durch feste oder variable Abtrennungen, beispielsweise Wände, Vorhänge oder dgl., im Wesentlichen voneinander getrennt und in sich geschlossen sind oder durch einzelne Zeltaufbauten oder dgl. realisiert sein.

Während der Behandlung des Materials wird die Luft des Raumes umgewälzt und gegebenenfalls befeuchtet und/oder mit Zuschlagstoffen versehen. Die Umwälzung der Luft des Raumes erfolgt durch eine von außen in den Raum eingebrachte Zuluft, wobei gleichzeitig Luft des Raumes abgeführt wird und somit eine Durchlüftung des Raumes durch Verdrängung von Raumluft durch die Zuluft erfolgt. In der Halle wird dabei bevorzugt kaum Überoder Unterdruck aufgebaut. Die durch Verdrängung nach außen abgeführte Abluft stellt vorzugsweise hauptsächlich eine mit aufgewirbelten Partikeln des Materials bzw. Dampf kontaminierte Raumluft dar.

Zur Zufuhr von Luft von außerhalb in den Raum und/oder zur Abfuhr von Luft des Raumes nach außen sind mehrere Luftdurchlässe 24 angeordnet. Die Luftdurchlässe 24 können ein Teil der vorstehend beschriebenen Absaugung 15 sein bzw. durch die Zuluftöffnungen 14 gebildet sein. Es ist möglich, dass die Luftdurchlässe 24 je nach Ansteuerung bzw. Modus das Einbringen von Zuluft und auch das Abführen von Abluft ermöglichen, öder je eigene Luftdurchlässe 24 zum Einbringen von Zuluft und eigene Luftdurchlässe 24 zum Abführen von Abluft vorgesehen sind. Zweckmäßiger Weise kann die Ansteuerung der Luftdurchlässe 24 durch die Regelungseinrichtung 12 erfolgen, sodass eine zentrale Steuerung des Verfahrens erfolgt. Ebenso besteht jedoch die Möglichkeit einer manuellen Steuerung bzw. Regelung der Funktion der Luftdurchlässe 24 durch eine Bedienperson.

Die Behandlung des Materials erfolgt über ein Umwälzgerät 25, das sich in eine durch einen Pfeil dargestellte Umwälzrichtung 26 bewegt. Dieses Umwälzgerät 25 weist eine entsprechende Einrichtung zum Umwälzen bzw. Umverteilen des Materials einer zu bearbeitenden Kompostmiete 5 auf, wobei durch das Umwälzen zwangsläufig Bestandteile des Materials aufgewirbelt werden und zumindest teilweise in Form von Schwebepartikeln in der Luft verbleiben. Jener Bereich des Raumes, der diese mit Schmutzpartikel bzw. mit Wasserdampf kontaminierte Luft aufweist, liegt üblicherweise in einer von der Umwälzrichtung 26 abgewandten Hinterseite 27 des Umwälzgerätes 25. Um eine Verteilung und Ausbreitung der kontaminierten Luft über weitere Bereich des Raumes zu verhindern, insbesondere Bewegung der kontaminierten Luft in Richtung der Umwälzrichtung 26 zu verhindern, wird nunmehr im Bereich einer Vorderseite 28, der in Umwälzrichtung 26 vor dem Umwälzgerät 25 liegt und/oder in einem oberhalb des Umwälzgerätes 25 liegenden Bereich, Zuluft durch die Luftdurchlässe 24 eingebracht und im Bereich der Hinterseite 27 des Umwälzgerätes 25 Luft des Raumes abgeführt.

Wie in Fig. 2 schematisch durch einen Pfeil 29 dargestellt, wird im Bereich der Vorderseite 28 Zuluft in die Halle 1 eingeströmt und, durch einen weiteren Pfeil 30 symbolisiert, in einem Bereich der Hinterseite 27 ein Abluftstrom aus der Halle abgeführt, d.h. es wird Raumluft in der Halle 1 durch Zuluft verdrängt. Der durch die Pfeile 29 und 30 symbolisierte Luftstrom ist gegen die Umwälzrichtung 26 gerichtet, um den Raum im Bereich der Vorderseite 28 des Umwälzgerätes 25 von Verschmutzung bzw. Nebel freizuhalten.

Die Luftdurchlässe 24 können an sich gegenüberliegenden Endbereichen des Raumes der Halle 1 angeordnet sein, insbesondere an Stirnseiten 2 der Halle 1, wobei an einem Endbereich des Raumes Zuluft eingebracht wird und am gegenüberliegenden Endbereich des Raumes Abluft abgesaugt wird. Mit einem derartigen Aufbau lässt sich in einfacher Weise ein über die ganze Hallenlänge ziehender, die kontaminierte Luft verdrängender Luftstrom erzeugen.

Eine weitere, anhand der in Fig. 2 links außen dargestellten Halle 1 ersichtliche Möglichkeit besteht darin, dass die Zufuhr und/oder Abfuhr von Luft des Raumes durch mehrere, entlang der Längsrichtung des Raumes bzw. der Halle 1 voneinander distanzierte und oberhalb der Kompostmieten 5 angeordnete Luftdurchlässe 24 erfolgt. Hierzu kann ein in Längsrichtung der Halle 1 verlaufendes Lüftungsrohr oberhalb der Mieten 5 angeordnet sein, insbesondere an Oberseite der Halle 1 aufgehängt oder außen am Hallendach verlaufend angeordnet sein, dass über dessen Längsverlauf mehrere voneinander beabstandete Luftdurchlässe 24 aufweist und das in die Außenumgebung der Halle 1 oder eine Abluftbehandlungsanlage mündet. Die Luftdurchlässe 24 können hierbei in einer Reihe über den Längsverlauf der Halle 1 angeordnet sein und separat oder in Gruppen aktiviert oder deaktiviert werden. Insbesondere können die Luftdurchlässe 24 in den hinter dem Umwälzgerät 25 liegenden Bereich zur Absaugung von Abluft aktiviert sein. Die Luftdwchlässe 24 im vor dem Umwälzgerät 25 liegenden Bereich können gegebenenfalls gleichzeitig zur Luftzufuhr aktiviert sein.

Die Zuluft kann von der Außenumgebung ohne Vorbehandlung über die Luftdurchlässe 24 ins Innere der Halle 1 transferiert werden und es kann über weitere Luftdurchlässe 24 diese wiederum direkt in die Außenumgebung freigesetzt werden, wobei die Luftdurchlässe 24 zur Abfuhr von Abluft eine Behandlungsstufe aufweisen können, z.B. eine chemische Behandlungsstufe mit Zerstäuber oder dgl., mit der die Abluft behandelt und von Geruchs- und Staubpartikeln befreit werden kann, wie dieses Prinzip bereits für die Absaugung 15 beschrieben wurde.

Die Luftdurchlässe 24 können an ein Leitungssystem 31 gebunden sein, wobei der Transportweg der Abluft bzw. Zuluft über das Leitungssystem 31 kontrolliert werden kann. Zum besseren Verständnis ist das Leitungssystem in der Fig. 2 in eine Zuleitung 32 und eine Ableitung 33 aufgeteilt, wobei die Ableitung 33 ausgangsseitig in eine Abluftbehandlungsanlage 34 mündet und die Zuleitung 32 eingangsseitig mit Zuluft von Außen gespeist wird. Die Zu- und Ableitungen 32, 33 können selbstverständlich in einer Leitung kombiniert sein bzw. können diese bei Bedarf die umgekehrte Funktionsweise aufweisen.

Im Allgemeinen sei zu den Luftdurchlässen 24 angemerkt, dass diese vorzugsweise in Strömungsvolumen und/oder der Förderrichtung regulierbar sind und beispielsweise durch Öffnungen wie z.B. querschnittsveränderliche Schlitz- oder Schachtöffnungen bzw. Fenster oder Druckerzeuger in Form von Ventilatoren, Kompressoren, Gebläse oder dgl. gebildet sind. Derartige Vorrichtungen sind dem Fachmann aus dem Stand der Technik bekannt, weshalb an dieser Stelle nicht näher auf diese eingegangen wird.

Über das mit den Luftdurchlässen 24 verbundene Leitungssystem 31 ist nun ein vorzugsweise durch die Regelungseinrichtung 12 bestimmter Transfer von Abluft aus der entsprechenden Halle 1 zu einer Abluftbehandlungsanlage 34 möglich. Die Zuleitungen 32 und Ableitungen 33 des Leitungssystems 31 können beispielsweise durch Rohrleitungen zur Förderung von Gasen gebildet sein. Die Abluftbehandlungsanlage 34 ist vorzugsweise durch den Bio-filter 9 oder einem Wäscher gebildet, wobei jede beliebige Einrichtung zur Abscheidung bzw. Filterung von Partikeln aus Gasen verwendet werden kann.

Die Luftumwälzung im Raum der Halle 1 kann derart erfolgen, dass durch Einbringung von zusätzlicher Zulüft über einen der Luftdurchlässe 24 oder durch Absaugen von Abluft über einen der Luftdurchlässe 24 die Luft des Raumes in Richtung der die Abluft abführenden Ableitung 33 in Bewegung gesetzt wird und von der kontaminierten Luft die Abluftbehandlungsanlage 34 zur Reinigung durchlaufen wird, worauf die gereinigte Luft in die Außenumgebung freigesetzt wird.

Eine weitere Möglichkeit der Luftumwälzung in der Halle 1 besteht darin, dass nur ein Teil der Abluft der Abluftbehandlungsanlage 34 zugeführt wird und der restliche Anteil des Absaugvolumens in eine der Hallen 1 oder eine Pufferstation zurückgespeist wird. Die Abluft wird dabei vorzugsweise in eine Halle 1 zurückgespeist, in der zu diesem Zeitpunkt keine Behandlung von Material durch Umwälzen stattfindet. Die Verunreinigungen der in die Halle(n) 1 zurückgespeisten Abluft können sich dabei am Hallenboden 4 bzw. den Kompostmieten 5 absetzen.

In das Leitungssystem 31 kann ein Strömungserzeuger, insbesondere ein Verdichter 35, geschalte: sein, über den das gewünschte Volumen an Abluft der Abluftbehandlungsanlage 34 zugeführt wird. Die der Abluftbehandlungsanlage 34 zuzuführende Abluftmenge kann somit über Steuerung des Verdichters 35 bestimmt werden. Dies ist deshalb von Vorteil, da die Abluftbehandlungsanlage 34 gezielt nur mit einer bestimmten Kapazität an Abluft belastet werden kann und beispielsweise die in dieser befindlichen Filter wesentlich weniger gewartet bzw. ausgetauscht werden müssen.

Weiters können ein oder mehrere Wärmetauscher 36 im Leitungssystem 31 angeordnet sein, sodass zumindest einige der Luftdurchlässe 24 über die Ableitung 33 und gegebenenfalls Zuleitung 32 zum Wärmetauscher 36 geführt sind. Die über die Luftdurchlässe 24 aus dem Raum der Halle 1 abgeführte und üblicherweise warme Abluft kann vor dem Einblasen in die Abluftbehandlungsanlage 34 die vorhandene Wärmeenergie an den Wärmetauschern 36 abgeben. Diese Wärmeenergie kann beispielsweise zum Vorwärmen der neu in die Halle 1 einzubringenden Zuluft genutzt werden, wodurch Nebelbildung in der Halle verhindert wird oder für andere Zwecke, z.B. zum Heizen von umliegenden Gebäuden bzw. der Steuerzentrale 13 oder dgl. genutzt werden.

Im Allgemeinen sei angemerkt, dass die Erzeugung der Luftströmung im Innenraum der Halle 1 bevorzugt nach dem Verdrängungsprinzip erfolgt, d.h. verbrauchte bzw. kontaminierte Raumluft durch von außen eingebrachte Zuluft verdrängt wird. Hierzu ist pro Halle 1 zumindest einer der Luftdurchlässe 24 zur Förderung von Zuluft ausgebildet und zumindest ein weiterer Luftdurchlass 24 zur Abfuhr oder Absaugung von Abluft vorgesehen. Die Luftströmung kann durch Förderung von Luft durch die Luftdurchlässe 24 erzeugt werden oder durch einblasen oder ansaugen von einem im Leitungssystem 31 angeordneten Strömungserzeuger, z.B. dem Verdichter 35, wobei in diesem Fall die entsprechenden Zu- bzw. Ableitungen 22, 33 gesperrt oder freigegeben werden können.

Zum Aufbau der Hallen 1 sei angemerkt, dass an den sich gegenüberliegenden Stirnseiten 2 der Halle 1 bevorzugt jeweils ein Tor, insbesondere Rolltor 3, in der Stirnwand 2 angeordnet ist und die Halle 1 somit von beiden Stirnseiten zugänglich ist. Um die Abluftmenge möglichst gering zu halten, ist der Querschnitt, d.h. das Breiten- zu Höhenverhältnis der Halle 1, von Bedeutung, wobei entsprechend den Querschnittsabmessungen der Halle 1 die Fördermenge durch die Luftdurchlässe 24 bzw. den Verdichter 35 und die Abluftbehandlungsanlage 34 bestimmt ist. Je nach Länge der Halle 1 ist gegebenenfalls vor Beginn der Behandlung des Materials eine gewisse Vorlaufzeit einzuhalten, in der ein durch die Hallenlänge verlaufender Luftzug erzeugt wird.

Abschließend sei angemerkt, dass die Steuerung bzw. Regelung der einzelnen Komponenten, wie der Luftdurchlässe 24, der Abluftbehandlungsanlage 34, des Verdichters 35, der Beregnungsvorrichtung 10 des Abluftgebläses 8, usw. durch die Regelungseinrichtung 12 automatisiert über entsprechende Steuerprogramme, insbesondere mittels eines computerisierten Systems, welches Programmlogiken verarbeitet, oder dgl., erfolgen, wobei selbstverständlich auch ein manuelles Eingreifen über entsprechende Steuermittel möglich ist. Beispielsweise kann eine Bedienperson, insbesondere ein Fahrer des Umwälzgerätes, mittels eines drahtlos mit den Luftdurchlässen 24 bzw. der Regelungseinrichtung 12 oder der Empfangs-Sendeeinrichtung 23 kommunizierenden Steuergerätes, wie. z.B. einer Funkfernsteuerung oder dgl., die Richtung der Luftströmung in der Halle 1 festlegen.

Der Ordnung halber sei abschließend darauf hingewiesen, dass zum besseren Verständnis der erfindungsgemäßen Vorrichtung Bestandteile teilweise unmaßstäblich und/oder vergrößert und/oder verkleinert dargestellt wurden.

Die den eigenständigen erfinderischen Lösungen zugrundeliegende Aufgabe kann der Beschreibung entnommen werden.

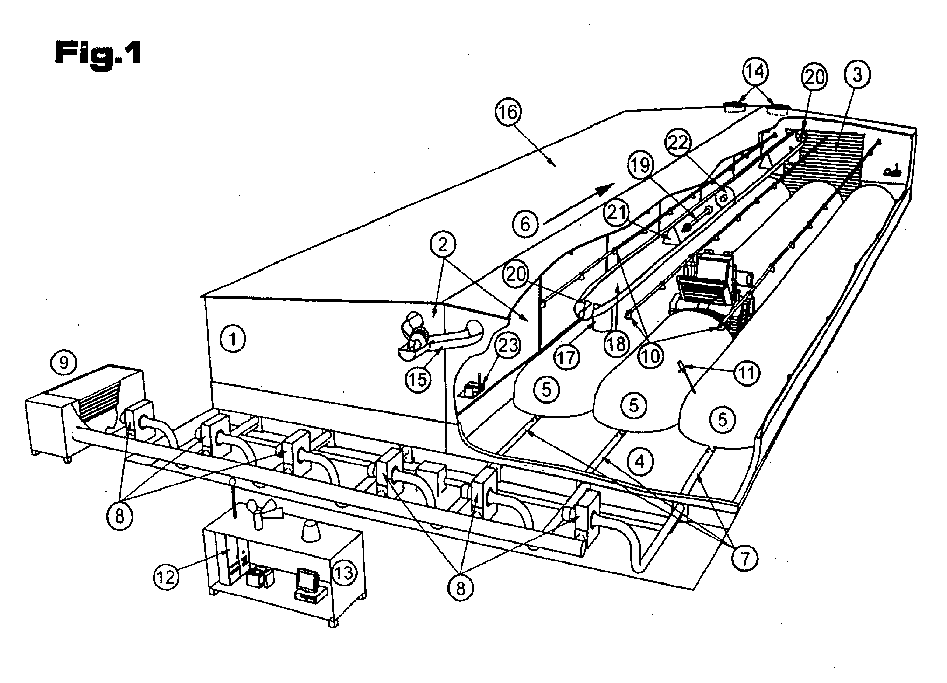

Vor allem können die einzelnen in den Fig. 1; 2 gezeigten Ausführungen den Gegenstand von eigenständigen erfindungsgemäßen Lösungen bilden. Die diesbezüglichen erfindungsgemäßen Aufgaben und Lösungen sind den Detailbeschreibungen dieser Figuren zu entnehmen.

### Bezugszeichenaufstellung

- 1: Halle
- 2: Stirnseite der Halle 1
- 3: Rolltor in der Stirnwand 2 der Halle 1
- 4: Hallenboden
- 5: Miete

- 6: Längsrichtung der Halle 1
- 7: Abluftkanal im Hallenboden 4
- 8: Abluftgebläse
- 9: Biofilter
- 10: Beregnungsvorrichtung

- 11: Temperatur- oder chemischer Fühler für eine Miete 5
- 12: Regelungseinrichtung
- 13: Steuerzentrale
- 14: Zuluftöffnung für die Halle 1
- 15: Absaugung der Hallenluft

- 16: Hallendach
- 17: Umlufteinrichtung
- 18: Umluftrohr der Umlufteinrichtung 17
- 19: Umluftrichtung
- 20: Förderlüftersatz

- 21: Beregner
- 22: Zerstäuber
- 23: Empfangs-Sendeeinrichtung der Regelungseinrichtung 12
- 24: Luftdurchlass
- 25: Umwälzgerät

- 26: Umwälzrichtung (Pfeil)
- 27: Hinterseite
- 28: Vorderseite
- 29: Pfeil
- 30: Pfeil

- 31: Leitungssystem
- 32: Zuleitung
- 33: Ableitung
- 34: Abluftbehandlungsanlage
- 35: Verdichter
- 36: Wärmetauscher

## Patentansprüche

1. Verfahren zur biologisch-mechanischen Behandlung von organischem Material mit Absaugung von Abluft unterhalb des Materials, wobei die Behandlung in einem im Wesentlichen geschlossenen Raum erfolgt, bei der die Absaugung von Abluft zur Kühlung oder Herabsetzung von chemischen Parametern auf jenen Bereich beschränkt ist, in dem eine Überschreitung von Funkmeldern an eine Regeleinrichtung (12) gemeldet wird, **dadurch gekennzeichnet, dass** während der Behandlung durch Umsetzen oder dgl. die Luft des Raumes umgewälzt, befeuchtet und mit Zuschlagstoffen versetzt wird und durch entstehenden Unterdruck die Nebelbildung verhindert wird und die Umwälzrichtung für die Behandlung ausgewählt wird.

2. Verfahren nach Anspruch 1, **dadurch gekennzeichnet, dass** die erforderliche Feuchtigkeit mit Beregnung hergestellt wird.

3. Verfahren nach Anspruch 1 oder 2, **dadurch gekennzeichnet, dass** bei Überschreiten von Grenzwerten die Luft im Raum durch Zuluft von außen ersetzt wird und die Abluft durch eine chemische Nachbehandlung von Geruchs- und Staubbelastung befreit wird, wobei die Steuerung von außen erfolgt.

4. Verfahen nach einem der Ansprüche 1 bis 3, durch gekennzeichnet, dass zur Umwälzung der Luft des Raumes eine Zuluft von außen eingebracht wird und gleichzeitig Luft des Raumes, insbesondere mit Partikeln bzw. Dampf kontaminierte Raumluft, abgeführt wird.

5. Verfahren nach einem der Ansprüche 1 bis 4, **dadurch gekennzeichnet, dass** in einem oberhalb bzw. in einer Umwälzrichtung vor einem Umwälzgerät (25) für das Material liegenden Bereich des Raumes Zuluft eingebracht wird und in einem hinter dem Umwälzgerät (25) liegenden Bereich des Raumes Abluft aus dem Raum abgeführt wird.

6. Verfahren nach einem der Ansprüche 1 bis 5, **dadurch gekennzeichnet, dass** zur Umwälzung der Luft im Raum ein Luftstrom erzeugt wird, der im Wesentlichen entgegen der Umwälzrichtung des Umwälzgeräts (25) gerichtet ist.

7. Verfahren nach einem der Ansprüche 1 bis 6, **dadurch gekennzeichnet, dass** die Zufuhr und/oder Abfuhr von Luft des Raumes über an gegenüberliegenden Endbereichen des Raumes, insbesondere Stirnseiten (2) einer Halle (1), vorgesehene Luftdurchlässe (24) erfolgt.

8. Verfahren nach einem der Ansprüche 1 bis 6, **dadurch gekennzeichnet, dass** die Zufuhr und/oder Abfuhr von Luft des Raumes durch einen oder mehrere entlang der Längsrichtung des Raumes voneinander distanzierte und oberhalb des Materials, insbesondere an einer Oberseite der Halle (1), angeordnete Luftdurchlässe (24) erfolgt.

9. Verfahren nach einem der Ansprüche 1 bis 8, **dadurch gekennzeichnet, dass** die aus dem Raum abgeführte, warme Abluft einem Wärmetauscher (36) zugeführt wird und gegebenenfalls die dem Raum zugeführte Zuluft durch den bzw. einen weiteren Wärmetauscher (36) vorgewärmt wird.

10. Verfahren nach einem der Ansprüche 1 bis 9, **dadurch gekennzeichnet, dass** die Umwälzrichtung für die Behandlung ausgewählt wird und Luftdurchlässe (24) für die Zufuhr und Abfuhr von Luft des Raumes in Abhängigkeit der Umwälzrichtung gesteuert bzw. geregelt werden.

11. Verfahren nach einem der Ansprüche 1 bis 10, **dadurch gekennzeichnet, dass** zumindest ein Teil des Volumens der Abluft des Raumes durch eine Abluftbehandlungsanlage (34) gereinigt wird.

12. Verfahren nach einem der Ansprüche 1 bis 11, **dadurch gekennzeichnet, dass** ein Anteil des Volumens der aus dem Raum abgeführten Abluft unbehandelt in den Raum oder einen bzw. mehrere andere derartiger Räume, wo eine Behandlung von Material zu diesem Zeitpunkt bevorzugt nicht stattfindet, zurückgespeist wird.

13. Verfahren nach Anspruch 4, **dadurch gekennzeichnet, dass** die Absaugung von Abluft zur Kühlung oder Herabsetzung von chemischen Parametern auf jenen Bereich beschränkt ist, in dem eine Überschreitung von Funkmeldern an eine Regeleinrichtung (12) gemeldet wird.

14. Vorrichtung zur Durchführung des Verfahrens nach einem der Ansprüche 1 bis 3, wobei der Raum sich in einer im Wesentlichen geschlossenen Halle (1) befindet mit einem Hallenboden (4), auf dem das organische Material aufgeschichtet ist und in den Abluftkanäle (7), parallel zueinander, eingelassen sind, von denen jeder mit einem gesonderten Abluftgebläse (8) verbunden ist, die miteinander in einen Biofilter (9) münden, wobei jedem Abluftkanal (7) ein Temperatur- oder Abluftstofffülhler (11) zugeordnet ist, der über Funk mit einer Empfangs- und Sendeeinrichtung (23) einer Regelungseinrichtung (12) in einer außerhalb liegenden Steuerzentrale (13) verbunden ist, die bei Messwertüberschreitung das zugeordnete Abluftgebläse (8) einschaltet, **dadurch gekennzeichnet, dass** Luftdurchlässe (24), die zur Zufuhr von Luft von außerhalb in den Raum und/oder zur Abfuhr von Luft des Raumes ausgebildet sind, angeordnet sind, wobei an sich gegenüberliegenden Endbereichen des Raumes, insbesondere an sich gegenüberliegenden Stirnseiten (2) der Halle (1), jeweils zumindest ein Luftdurchlass (24) angeordnet ist und/oder in einem oberhalb des Materials, insbesondere an einer Oberseite der Halle (1), liegenden Bereich mehrere, in Längsrichtung voneinander distanzierte Luftdurchlässe (24) angeordnet sind, und dass die Luftdurchlässe (24) über ein Leitungssystem (31) mit einer Abluftbehandlungsanlage (34), insbesondere dem Biofilter (9) strömungsverbunden sind und der Abluftbehandlungsanlage (34) ein das zu behandelnde Abluftvolumen bestimmender Verdichter (35) vorgeordnet ist.

15. Vorrichtung nach Anspruch 14, **dadurch gekennzeichnet, dass** in der Längsrichtung der Abluftkanäle (7) oberhalb des Materials eine Umlufteinrichtung (17) für das Umsetzen vorgesehen ist, mit einem Umluftrohr (18) großen Lumens, in dem die Hallenluft beim Umsetzen in eine von zwei Umluftrichtungen (19) mit einem der beiden endständig angebrachten Förderlüftersätze (20) mit axialem und/oder nach unten gerichteten Austritt befördert wird und deren Umluftrichtungen (19) gegen die Umsetzrichtung anpassbar sind.

16. Vorrichtung nach Anspruch 15, **dadurch gekennzeichnet, dass** im Umluftrohr (18) ein mittig angeordneter Zerstäuber (22) für Zuschlagstoffe zur Geruchs- und Staubbindung angebracht ist, dem wenigsten ein Beregner (21) in jeder Richtung vorgeordnet ist, wobei diese nicht oder nur einer oder beide zuschaltbar sind.

17. Vorrichtung nach Anspruch 14, **dadurch gekennzeichnet, dass** eine Beregnungsvorrichtung (10) vorgesehen ist, die vorzugsweise gesondert zuschaltbar ist

18. Vorrichtung nach Anspruch 14, **dadurch gekennzeichnet, dass** eine Absaugung (15) der Hallenluft nach außen vorgesehen ist, die an einer Stirnseite (2) der Halle (1) angeordnet ist, der von der anderen Stirnseite (2) von außen Frischluft zugeführt wird, um die Halle (1) zu durchlüften und damit die Geruchs- und Staubanteile in der Abluft zu vermindern, wobei zur Behandlung der ausgeblasenen Hallenluft der Absaugung (15) unmittelbar nachgeschaltet ein Zerstäuber für den Zusatz von Zuschlagstoffen angebracht ist.

19. Vorrichtung nach einem der Ansprüche 14 bis 18, **dadurch gekennzeichnet, dass** das biologische Material in Form von Mieten (5) dicht an dicht in Richtung der Abluftkanäle (7) verlaufend aufgesetzt wird.

20. Vorrichtung nach einem der Ansprüche 14 bis 19, **dadurch gekennzeichnet, dass** die in dem oberhalb des Materials liegenden Bereich angeordneten Luftdurchlässe (24) an einem bevorzugt in Hallenlängsrichtung verlaufenden und nach außen führenden Lüftungsrohr angeordnet sind.

21. Vorrichtung nach einem der Ansprüche 14 bis 20, **dadurch gekennzeichnet, dass** die Luftdurchlässe (24) durch vorzugsweise im Strömungsvolumen regulierbare Öffnungen, insbesondere Schlitz- oder Schachtöffnungen, oder durch Druckerzeuger, insbesondere, Ventilatoren, Kompressoren, Gebläse oder dgl., gebildet sind.

22. Vorrichtung nach einem der Ansprüche 14 bis 21, **dadurch gekennzeichnet, dass** zumindest ein Wärmetauscher (36) angeordnet ist, der mit zumindest einigen der Luftdurchlässe (24) über das Leitungssystem (31) in Wirkungsverbindung steht oder schaltbar ist.

23. Vorrichtung nach einem der Ansprüche 14 bis 22, **dadurch gekennzeichnet, dass** die Luftdurchlässe (24) zu deren Steuerung bzw. Regelung mit der Regelungseinrichtung (12) wirkungsverbunden sind.

24. Vorrichtung nach einem der Ansprüche 14 bis 23, **dadurch gekennzeichnet, dass** die Luftdurchlässe (24) über das Leitungssystem (31) mit weiteren Luftdurchlässen (24) von anderen, diskreten Räumen von Hallen (1) strömungsverbunden sind.

25. Vorrichtung nach einem der Ansprüche 14 bis 24, **dadurch gekennzeichnet, dass** die Vorrichtung zur Durchführung des Verfahrens nach einem der Ansprüche 4 bis 13 ausgebildet ist.

## Claims

1. Method for the biological-mechanical processing of organic material with a suction system underneath the material for extracting air, whereby processing takes place in an essentially closed room and the extraction of air for cooling or reducing chemical parameters is limited to the region in which exceeded limits are signalled to a control system (12) by radio detectors, **characterised in that** during processing, by turning or similar, the air in the room is circulated, moistened and admixed with additives and the formation of mist is prevented by the resultant negative pressure and the circulation direction for processing is selected.

2. Method as claimed in claim 1, **characterised in that** the moisture needed is generated by sprinkling.

3. Method as claimed in claim 1 or 2, **characterised in that** when threshold values are exceeded, the air in the room is replaced by feeding in air from outside and dust and odours are removed from the extracted air by a subsequent chemical treatment, the control being operated from outside.

4. Method as claimed in one of claims 1 to 3, **characterised in that**, in order to circulate the air in the room, air is fed in from outside and air, in particular air in the room contaminated with particles and steam, is extracted from the room.

5. Method as claimed in one of claims 1 to 4, **characterised in that** air is fed into a region of the room lying above or in a circulating direction upstream of a circulating device (25) for the material and air is extracted from the room in a region lying downstream of the circulating device (25).

6. Method as claimed in one of claims 1 to 5, **characterised in that**, in order to circulate the air in the room, an air flow is generated which is directed essentially opposite the circulating direction of the circulating device (25).

7. Method as claimed in one of claims 1 to 6, **characterised in that** air is fed into and/or extracted from the room via air vents (24) disposed at oppositely lying end regions of the room, in particular end faces (2) of a building (1).

8. Method as claimed in one of claims 1 to 6, **characterised in that** air is fed into and/or extracted from the room via one or more air vents (24) disposed along the longitudinal direction of the room, spaced at a distance apart from one another and above the material, in particular on a top face of the building (1).

9. Method as claimed in one of claims 1 to 8, **characterised in that** the warm air extracted from the room is fed to a heat exchanger (36) and the air fed into the room is optionally pre-heated in the or another heat exchanger (36).

10. Method as claimed in one of claims 1 to 9, **characterised in that** the circulating direction for the processing operation is selected and air vents (24) for feeding in and extracting air from the room are controlled and regulated as a function of the circulating direction.

11. Method as claimed in one of claims 1 to 10, **characterised in that** at least a part of the volume of air extracted from the room is cleaned by an extracted air treatment plant (34).

12. Method as claimed in one of claims 1 to 11, **characterised in that** a proportion of the volume of extracted air fed out of the room is fed back into the room untreated or to one or more other such rooms where material is preferably not being treated at this point in time.

13. Method as claimed in claim 4, **characterised in that** the extraction of air for cooling or reducing chemical parameters is restricted to the region where exceeded limits are signalled to a control system (12) by radio detectors.

14. Apparatus for implementing the method as claimed in one of claims 1 to 3, whereby the room is disposed in an essentially closed building (1) with a building floor (4) on which the organic material is layered and in which mutually parallel extraction air passages (7) are disposed, each of which is connected to a separate extractor fan (8), and which jointly open into a bio-filter (9), and every extraction air passage (7) co-operates with a temperature or extraction air sensor (1) connected via radio to a receiver and transmitter unit (23) of a control system (12) in an externally disposed central control system (13) which switches the co-operating extractor fan (8) on when measurement values are exceeded, **characterised in that** air vents (24) configured to feed air into the room from outside and/or extract air from the room are provided, and at least one air vent (24) respectively is disposed at oppositely lying end regions of the room, in particular at oppositely lying end faces (2) of the building (1), and/or several air vents (24) are disposed in a region lying above the material, in particular in a top face of the building (1), spaced at a distance apart from one another in the longitudinal direction, and the air vents (24) are connected via a pipe system (31) to establish a flow to an extracted air treatment plant (34), in particular the bio-filter (9), and a condenser (35) for determining the volume of extracted air to be treated is disposed upstream of the extracted air treatment plant (34).

15. Apparatus as claimed in claim 14, **characterised in that** a circulating device (17) is provided above the material in the longitudinal direction of the extraction air passages (7) for circulation purposes, with a circulation air pipe (18) with a large opening in which the building air is conveyed in one of two circulating directions (19) by one of the two independently mounted sets of conveyer fans (20) with an axially and/or downwardly directed outlet, and their circulation directions (19) opposite the conversion direction can be adapted.

16. Apparatus as claimed in claim 15, **characterised in that** a mister (22) for additives for binding odours and dust is mounted centrally in the circulation air pipe (18), upstream of which at least one sprinkler (21) is disposed, and the latter may not be connected, only one of them may connected or both may be connected.

17. Apparatus as claimed in claim 14, **characterised in that** a sprinkler device (10) is provided, which can preferably be connected separately.

18. Apparatus as claimed in claim 14, **characterised in that** a system for extracting (15) the building air to the outside is provided and is disposed in one end face (2) of the building (1) and fresh air is fed in from the other end face (2) in order to ventilate the building (1) and thus reduce the amount of odour and dust in the extracted air, and the extraction system (15) is mounted immediately downstream of a mister for feeding in additives in order to treat the air extracted from the building.

19. Apparatus as claimed in one of claims 14 to 18, **characterised in that** the biological material is placed in tightly packed heaps (5) extending in the direction of the extraction air passages (7).

20. Apparatus as claimed in one of claims 14 to 19, **characterised in that** the air vents (24) disposed in the region lying above the material are disposed in a venting pipe preferably extending in the building longitudinal direction and leading to the outside.

21. Apparatus as claimed in one of claims 14 to 20, **characterised in that** the air vents (24) are provided in the form of openings, the flow volume of which can preferably be regulated, in particular slot or shaft openings, or in the form of pressure generators, in particular ventilators, compressors, fans or similar.

22. Apparatus as claimed in one of claims 14 to 21, **characterised in that** at least one heat exchanger (36) is provided, which is or can be switched so as to establish an operating connection to at least some of the air vents (24) via the pipe system (31).

23. Apparatus as claimed in one of claims 14 to 22, **characterised in that** the air vents (24) are connected to the control system (12) during operation to enable them to be controlled and regulated.

24. Apparatus as claimed in one of claims 14 to 23, **characterised in that** the air vents (24) are connected via the pipe system (31) to establish a flow to other air vents (24) of other separate rooms of buildings (1).

25. Apparatus as claimed in one of claims 14 to 24, **characterised in that** the apparatus is configured to implement the method as claimed in one of claims 4 to 13.

## Revendications

1. Procédé pour le traitement bio-mécanique d'une matière organique avec aspiration de l'air d'évacuation situé en dessous de la matière, le traitement étant effectué dans un espace pour l'essentiel fermé, dans lequel l'aspiration de l'air d'évacuation, pour le refroidissement ou l'abaissement de paramètres chimiques, est limitée à la zone dans laquelle un dépassement est notifié par des radiomessageurs à un dispositif de régulation (12), **caractérisé en ce que**, pendant le traitement, l'air du local est mis en recirculation par transfert ou analogues, humidifié et additionné d'adjuvants, la dépression qui se forme empêchant la formation d'un brouillard, et le sens de la recirculation étant sélectionné pour le traitement.

2. Procédé selon la revendication 1, **caractérisé en ce que** l'humidité nécessaire est obtenue par arrosage.

3. Procédé selon la revendication 1 ou 2, **caractérisé en ce que**, lors du dépassement de valeurs limites, l'air se trouvant dans le local est remplacé par de l'air frais provenant de l'extérieur, et l'air d'évacuation est, par un traitement chimique subséquent, débarrassé de la charge d'odeur et de poussière, le pilotage étant réalisé de l'extérieur.

4. Procédé selon l'une des revendications 1 à 3, **caractérisé en ce que**, pour la recirculation de l'air du local, un air frais est introduit de l'extérieur, et simultanément l'air du local, en particulier l'air ambiant contaminé par des particules ou de la vapeur, est évacué.

5. Procédé selon l'une des revendications 1 à 4, **caractérisé en ce que**, dans une zone du local situé au-dessus d'un appareil de recirculation (25) du matériel, ou en amont de ce matériel dans un sens de recirculation, de l'air frais est introduit, et, dans une zone du local située en arrière de l'appareil de recirculation (25), l'air d'évacuation est évacué du local.

6. Procédé selon l'une des revendications 1 à 5, **caractérisé en ce que**, pour la recirculation de l'air dans le local, un courant d'air est produit, qui pour l'essentiel est dirigé contre le sens de recirculation de l'appareil de recirculation (25).

7. Procédé selon l'une des revendications 1 à 6, **caractérisé en ce que** l'amenée et/ou l'évacuation de l'air du local s'effectuent en passant par des traversées d'air (24), prévues dans des zones terminales opposées du local, en particulier les faces frontales (2) d'un hall (1).

8. Procédé selon l'une des revendications 1 à 6, **caractérisé en ce que** l'amenée et/ou l'évacuation de l'air du local s'effectuent par une ou plusieurs traversées d'air (24), disposées le long de la direction longitudinale du local, à distance les unes des autres, et au-dessus de la matière, en particulier sur une face supérieure du hall (1).

9. Procédé selon l'une des revendications 1 à 8, **caractérisé en ce que** l'air d'évacuation chaud évacué du local est amené à un échangeur de chaleur (36), et l'air frais amené dans le local est éventuellement préchauffé par le ou un autre échangeur de chaleur (36).

10. Procédé selon l'une des revendications 1 à 9, **caractérisé en ce que** le sens de recirculation est sélectionné pour le traitement, et les traversées d'air (24) sont pilotées ou régulées pour l'amenée et l'évacuation de l'air du local en fonction du sens de recirculation.

11. Procédé selon l'une des revendications 1 à 10, **caractérisé en ce qu'**au moins une partie du volume de l'air d'évacuation du local est purifiée par une installation (34) de traitement de l'air d'évacuation.

12. Procédé selon l'une des revendications 1 à 11, **caractérisé en ce qu'**une partie du volume de l'air d'évacuation évacué du local est renvoyée sans traitement dans le local ou dans un ou plusieurs locaux du type dans lequel un traitement de la matière à ce moment-là n'est de préférence pas effectué.

13. Procédé selon la revendication 4, **caractérisé en ce que** l'aspiration de l'air d'évacuation, pour le refroidissement ou la diminution de paramètres chimiques, est limité à la zone dans laquelle un dépassement est notifié par des radiomessageurs à un dispositif de régulation (12).

14. Dispositif pour la mise en oeuvre du procédé selon l'une des revendications 1 à 3, dans lequel le local se trouve dans un hall (1) pour l'essentiel fermé, comportant un sol de hall(4), sur lequel la matière organique est entassée, et dans lequel des gaines d'air d'évacuation (7) sont insérées parallèlement les unes aux autres, chaque gaine étant reliée à une soufflante d'air d'évacuation (8) distincte, les soufflantes débouchant toutes ensemble dans un biofiltre (9), un capteur (1) de température ou d'air d'évacuation (11) étant affecté à chaque gaine (7) d'air d'évacuation, capteur qui, par radio, est relié à un dispositif récepteur et humecteur (23) d'un dispositif de régulation (12) dans une centrale de commande (13) située à l'extérieur, qui en cas de dépassement des valeurs de mesure active la soufflante d'air d'évacuation (8) correspondante, **caractérisé en ce que** des traversées d'air (24) sont disposées, qui sont configurées pour l'amenée de l'air à partir de l'extérieur dans le local et/ou pour l'évacuation de l'air du local ; au moins une traversée d'air (24) étant disposée contre chacune des zones terminales opposées du local, en particulier au niveau de chacune des faces frontales opposées (2) du hall (1), et/ou, dans une zone située au-dessus de la matière, en particulier au niveau d'une face supérieure du hall (1), plusieurs traversées d'air (24) sont disposées à une certaine distance l'une de l'autre dans la direction longitudinale ; et **en ce que** les traversées d'air (24) sont reliées d'une manière fluidique, par un système de conduites (31), à une installation (34) de traitement de l'air d'évacuation, en particulier le biofiltre (9), et un compresseur (35), définissant le volume d'air d'évacuation à traiter, étant disposé en amont de l'installation (34) de traitement de l'air d'évacuation.

15. Dispositif selon la revendication 14, **caractérisé en ce que**, dans la direction longitudinale des gaines d'air d'évacuation (7), au-dessus de la matière, il est prévu un dispositif de circulation d'air (17) pour le transfert, comportant un tuyau (18) de circulation d'air à gros calibre, dans lequel l'air du hall, lors du transfert de l'air, est refoulé dans l'une de deux directions de circulation d'air (19), avec un de deux groupes de ventilateurs refoulants (20) rapportés en bout, avec une sortie axiale et/ou une dirigée vers le bas, et dont les directions de circulation d'air (19) peuvent être adaptées en fonction de la direction du transfert de l'air.

16. Dispositif selon la revendication 15, **caractérisé en ce qu'**un pulvérisateur (22), disposé en position centrale, et destiné à des adjuvants pour la fixation des odeurs et des poussières, est rapporté dans le tuyau de circulation d'air (18), pulvérisateur en amont duquel au moins un système d'arrosage (21) est disposé, dans chaque direction, ces derniers n'étant pas raccordables, ou alors seulement l'un, ou les deux, pouvant être raccordables.

17. Dispositif selon la revendication 14, **caractérisé en ce qu'**un dispositif d'arrosage (10) est prévu, qui de préférence peut être raccordé d'une manière distincte.

18. Dispositif selon la revendication 14, **caractérisé en ce qu'**une aspiration (15) de l'air du hall (1) vers l'extérieur est prévue, qui est disposé sur un côté frontal (2) du hall (1), de l'air frais est amené de l'extérieur, par l'autre face frontale (2), pour aérer le hall (1) et ainsi diminuer les charges d'odeur et de poussière dans l'air d'évacuation, auquel cas, pour le traitement de l'air évacué du hall, un pulvérisateur, destiné à ajouter des adjuvants, est rapporté en étant disposé immédiatement en aval de l'aspiration (15).

19. Dispositif selon l'une des revendications 14 à 18, **caractérisé en ce que** la matière biologique est placée sous forme de tas (5) disposés en contact les uns avec les autres dans la direction des gaines d'air d'évacuation (7).

20. Dispositif selon l'une des revendications 14 à 19, **caractérisé en ce que** les traversées d'air (24) disposées dans la zone située au-dessus de la matière sont disposées contre un tuyau d'aération, de préférence courant dans la direction longitudinale du hall et allant vers l'extérieur.

21. Dispositif selon l'une des revendications 14 à 20, **caractérisé en ce que** les traversées d'air (24) sont formées par des ouvertures de préférence régulables en fonction du volume d'écoulement, en particulier des ouvertures à fente ou à puits, ou par des générateurs de pression, en particulier des ventilateurs, des compresseurs, des soufflantes ou analogues.

22. Dispositif selon l'une des revendications 14 à 21, **caractérisé en ce qu'**au moins un échangeur de chaleur (36) est disposé, qui est relié par liaison d'interaction à au moins quelques-unes des traversées d'air (24) par l'intermédiaire du réseau de conduites (31), ou peut être activé par ces derniers.

23. Dispositif selon l'une des revendications 14 à 22, **caractérisé en ce que** les traversées d'air (24) sont reliées par liaison d'interaction, pour permettre leur pilotage ou leur régulation, au dispositif de régulation (12).

24. Dispositif selon l'une des revendications 14 à 23, **caractérisé en ce que** les traversées d'air (24) sont reliées par liaison fluidique, par l'intermédiaire du réseau de conduites (31), à d'autres traversées d'air (24) appartenant à d'autres locaux discrets de halls (1).

25. Dispositif selon l'une des revendications 14 à 24, **caractérisé en ce que** le dispositif est configuré pour la mise en oeuvre du procédé selon l'une des revendications 4 à 13.
